(19) **Europäisches Patentamt / European Patent Office / Office européen des brevets**

(11) **EP 1 996 072 B1**

(12) **EUROPEAN PATENT SPECIFICATION**

(45) Date of publication and mention
of the grant of the patent:
**05.05.2010 Bulletin 2010/18**

(51) Int Cl.:
***A61B 5/026*** (2006.01)

(21) Application number: **07705219.9**

(22) Date of filing: **15.02.2007**

(86) International application number:
**PCT/GB2007/000535**

(87) International publication number:
**WO 2007/093804 (23.08.2007 Gazette 2007/34)**

(54) **PERIPHERAL ARTERIAL PATENCY MONITORING SYSTEM AND METHOD**

PERIPHERES ARTERIEN-DURCHGÄNGIGKEITSÜBERWACHUNGSSYSTEM UND
-VERFAHREN

SYSTEME ET PROCEDE POUR LA SURVEILLANCE DE L'ETAT DES ARTERIES PERIPHERIQUES

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **15.02.2006 GB 0603006**

(43) Date of publication of application:
**03.12.2008 Bulletin 2008/49**

(60) Divisional application:
**10158890.3**

(73) Proprietor: **Dialog Devices Limited
Loughborough
Leicestershire LE11 3EH (GB)**

(72) Inventors:
• **CRABTREE, Vincent
Leicestershire LE11 3TU (GB)**

• **The other inventor has agreed to waive his
entitlement to designation.**

(74) Representative: **Higgin, Paul
Swindell & Pearson Limited
48 Friar Gate
Derby DE1 1GY (GB)**

(56) References cited:
**EP-A1- 0 444 934     WO-A-2005/065533
US-A- 5 050 613**

• **JOHN ALLEN ET AL: "Photoplethysmography
detection of lower limb peripheral arterial
occlusive disease: a comparison of pulse timing,
amplitude and shape characteristics"
PHYSIOLOGICAL MEASUREMENT, INSTITUTE
OF PHYSICS PUBLISHING, BRISTOL, GB, vol. 26,
no. 5, 1 October 2005 (2005-10-01), pages 811-821,
XP020092231 ISSN: 0967-3334**

**Description**

FIELD OF THE INVENTION

Embodiments of the invention relate to assessing blood supply to a peripheral portion of an animal. In particular assessing the impairment of blood supply to a peripheral portion of an animal, such as the foot of a human.

BACKGROUND TO THE INVENTION

Peripheral Arterial Disease (PAD) is a subset of peripheral vascular disease, characterized by reduced arterial blood flow to the extremities. The most common early symptoms are pain, tingling or muscle cramps in the muscles of the leg on exercise. Later stage severe symptoms can be reduced ability to heal wounds, which can result in infections on the foot, gangrene or tissue death (necrosis).

A simple blood pressure measurement can sometimes be used to determine severity of disease, commonly called ABP or ABPI procedure, where the systemic upper body systolic blood pressure is compared to the systolic blood pressure determined from the arteries of the foot. If the foot systolic blood pressure is less than half the systemic systolic blood pressure or less than 40/50mmHg then it is likely that severe occlusive arterial disease is present, and the patient is in danger of infection and tissue necrosis.

Under these circumstances, surgical intervention is required. This was traditionally an arterial bypass graft, although trans-cutaneous endovascular angioplasty is becoming more common, whereby blockages are removed from inside the artery without surgery.

A typical peripheral arterial bypass graft procedure consists of locating the occluded artery, typically the popliteal artery around the knee, and bridging the occlusion with either an artificial bypass graft or a vein harvested from the patient.

After surgery, it is important to check that the bypass graft does not become blocked and is still open, or patent. This is traditionally performed in a manual way by nurses on regular intervals feeling for pulses or using handheld vascular Doppler wands. However, subjectivity and logistic problems can mean that a failing bypass graft is not detected until the blockage is significant or the artery is totally occluded. Failed grafts require an additional surgical re-intervention to replace the blocked graft, causing procedure costs to at least double.

Early detection of failing bypass grafts may be treatable via drug therapy, both oral and by intra-vascular injection.

It would be desirable to provide for post-operative monitoring of the patency of an artery.

An automatic device that monitors arterial blood supply to provide an indication of graft patency, signaling an alarm if the arterial blood supply ceases or drops below a given threshold, could be a useful clinical tool in the monitoring of immediate post operative peripheral bypass grafts.

A device that provides an indication of peripheral arterial blood supply perioperatively may also be useful to surgeons during the installation of a bypass graft, to assess the level of restored arterial blood supply. This approach may also find applications during percutaneous endovascular angioplasty to determine if the blockage or narrowing has been successfully obliterated and whether additional narrowing requires treatment to restore adequate circulation to the periphery.

The article by John Allen et al., "Photoplethysmography detection of lower limb peripheral arterial occlusive disease: a comparison of pulse timing, amplitude and shape characteristics", Physiological Measurement, Volume 26, Number 5, October 2005 , pp. 811-821, describes arterial patency monitoring, wherein photoplethysmography pulses are acquired simultaneously from the right and left leg and different pulse characteristics and bilateral differences are determined.

BRIEF DESCRIPTION OF THE INVENTION

The invention is defined in the appended claims.

According to some embodiments of the invention there is provided an arterial patency monitoring system for monitoring patency of an artery of a limb comprising: a first probe positioned adjacent a first tissue area of a limb for producing first output signals dependent upon a first arterial blood supply to the first tissue area; a second probe positioned adjacent a second different tissue area of the limb for producing second, different output signals dependent upon a second arterial blood supply to the second tissue area; and means for processing the first output signals to produce a first arterial blood supply metric for the first arterial blood supply and a second, different, arterial blood supply metric for the first arterial blood supply; means for processing the second output signals to produce the first arterial blood supply metric for the second arterial blood supply and the second arterial blood supply metric for the second arterial blood supply; means for using a predetermined function that takes as its arguments at least the first and second arterial blood supply metrics for the first arterial blood supply to produce a combined arterial patency metric for the first blood supply; means for using the predetermined function that takes as its arguments at least the first and second arterial blood supply metrics for the second arterial blood supply to produce a combined arterial patency metric for the second arterial blood supply; means for comparing the combined arterial patency metric for the first arterial blood supply with the combined arterial patency metric for the second arterial blood supply; and means for determining the patency of a first artery that provides the first arterial blood supply using a difference between the combined arterial patency metric for the first arterial blood supply and the combined arterial patency metric for the second arterial blood supply. The arterial patency monitoring system may be a postoperative arterial patentcy system. The arterial patency monitoring system may be a perioperative arterial patency system.

According to some embodiments of the invention there is provided a signal processing unit of an arterial patency monitoring system for monitoring patency of an artery of a limb, the unit comprising: a first input for receiving first input signals,from a first probe that are dependent upon a first arterial blood supply to a first tissue area of a limb; means for processing the first output signals to produce a first arterial blood supply metric for the first arterial blood supply and a second, different, arterial blood supply metric for the first arterial blood supply; means for using a predetermined function that takes as its arguments at least the first and second arterial blood supply metrics for the first arterial blood supply to produce a combined arterial patency metric for the first arterial blood supply; a second input for receiving second input signals from a second probe that are dependent upon a second arterial blood supply to a second tissue area of the limb; means for processing the second output signals to produce a first arterial blood supply metric for the second arterial blood supply and a second, different, arterial blood supply metric for the second arterial blood supply; means for using the predetermined function that takes as its arguments at least the first and second arterial blood supply metrics for the second arterial blood supply to produce a combined arterial patency metric for the second arterial blood supply; means for comparing the combined arterial patency metric for the first arterial blood supply with the combined arterial patency metric for the second arterial blood supply; and means for determining the patency of a first artery that provides the first arterial blood supply using a difference between the combined arterial patency metric for the first arterial blood supply and the combined arterial patency metric for the second arterial blood supply. The arterial patency monitoring system may be postoperative or perioperative.

According to some embodiments of the invention there is provided an arterial patency monitoring method for monitoring patency of an artery of a limb comprising: positioning a first probe adjacent a first tissue area of a limb fed by a first arterial blood supply such that it produces first output signals that are dependent upon the first arterial blood supply to the first tissue area; positioning a second probe adjacent a second tissue area of the limb fed by a second arterial blood supply such that it produces second output signals that are dependent upon the second arterial blood supply to the first tissue area; processing the first output signals to produce a first arterial blood supply metric for the first arterial blood supply and a second, different, arterial blood supply metric for the first arterial blood supply; combining at least the first and second arterial blood supply metrics for the first arterial blood supply, using a predetermined function that takes as its arguments at least the first and second arterial blood supply metrics for the first arterial blood supply, to produce a combined arterial patency metric for the first blood supply; processing the second output signals to produce a first arterial blood supply metric for the second arterial blood supply and a second, different, arterial blood supply metric for the second arterial blood supply; combining at least the first and second arterial blood supply metrics for the second arterial blood supply, using the predetermined function that takes as its arguments at least the first and second arterial blood supply metrics for the second arterial blood supply, to produce a combined arterial patency metric for the second arterial blood supply; comparing the combined arterial patency metric for the first arterial blood supply with the combined arterial patency metric for the second arterial blood supply; and determining the patency of a first artery that provides the first arterial blood supply using a difference between the combined arterial patency metric for the first arterial blood supply and the combined arterial patency metric for the second arterial blood supply. The arterial patency monitoring may be a postoperative and/or perioperative.

BRIEF DESCRIPTION OF THE DRAWINGS

For a better understanding of the present invention reference will now be made by way of example only to the accompanying drawings in which:

Fig 1 schematically illustrates a peripheral arterial patency monitor (PAPM);
Fig 2 illustrates an example of a probe positioning apparatus;
Fig 3 illustrates a typical arterial blood supply signal;
Fig 4 illustrates a power spectral density (PSD) of the arterial blood supply signal illustrated in Fig 4; and
Fig 5 illustrates the envelope of the arterial blood supply signal illustrated in Fig 3;
Figs 6, 7 and 8 each illustrate a different probe positioning apparatus; and
Fig 9 illustrates a probe comprising an arrangement of optical sensors and corresponding light sources.
Figs 10A and 10B schematically illustrate the same compact PAPM from different perspectives.

DETAILED DESCRIPTION OF EMBODIMENTS OF THE INVENTION

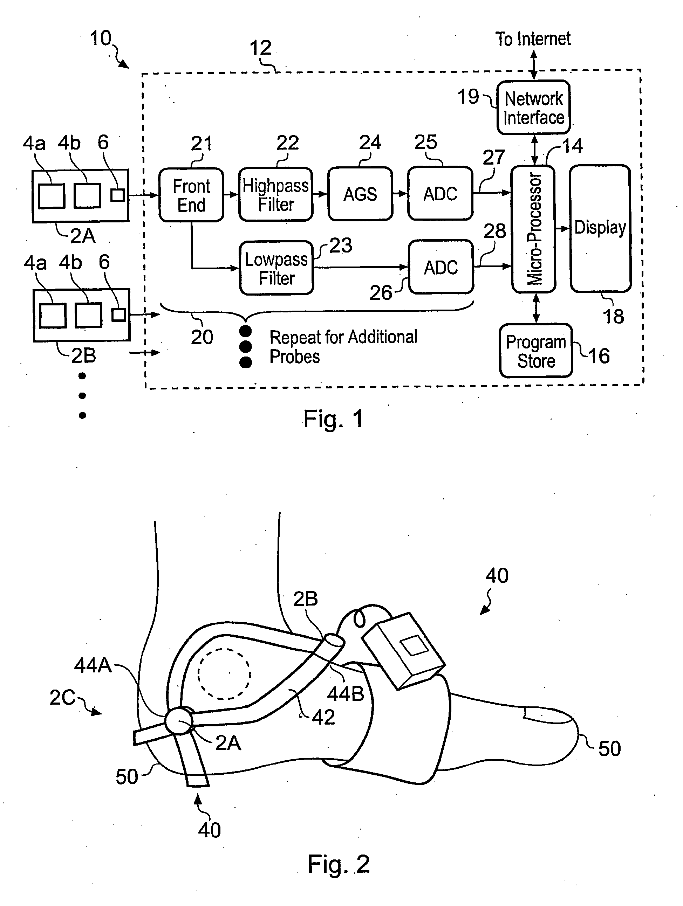

Figure 1 schematically illustrates a peripheral arterial patency monitor (PAPM) system 10. A peripheral arterial patency monitor has to solve specific problems that may be quite different to systemic monitors, such as pulse oximeters. A peripheral arterial patency monitor assesses the level of arterial blood supply to a distal peripheral location, typically the foot, but is not assessing life sustaining or critical functions.

The monitor 10 comprises: one or more probes 2 and a base station unit 12 which is arranged to receive input (s) from the probe(s) 2 and provide control output(s) to the probes(s) 2.

A probe positioning apparatus 40 is illustrated in Fig 2. This is used for accurately locating the probe(s) 2 on desired arteries at a distal peripheral part 50 of a subject, such as the foot.

A probe 2, in this example, comprises a sensor or sensors 4 for measuring a physiological-dependent parameter or parameters related to peripheral arterial blood supply. These parameters may for example include one or more of: arterial blood supply strength, arterial blood supply caliber, peripheral pulse rate, skin color redness, and skin temperature.

One of the sensors 4 is a plethysmograph sensor that measures peripheral arterial blood supply. Another of the sensors 4 may be a temperature sensor.

A strain-gauge plethysmograph or impedance plethysmograph may be used as a plethysmograph sensor 4 to assess the peripheral arterial blood supply. Such plethysmographs provide a signal that can be processed by the base unit 12 to provide an indication of arterial blood supply strength, arterial blood supply caliber and peripheral pulse rate.

Impedance plethysmography may typically employ multiple probes with a time division multiplex scheme or a frequency division multiplex scheme, to avoid probe interference. A two-part large area probe can be used on opposite sides of the foot to provide an integrated signal corresponding to the average arterial supply to the foot. An optical sensor 4 would additionally be required to assess skin color redness (if required).

An optical plethysmograph may also be used as the plethysmograph sensor 4 to assess the peripheral arterial blood supply. Such a plethysmograph also provides a signal that can be processed by the base unit 12 to provide an indication of arterial blood supply strength, arterial blood supply caliber and peripheral pulse rate. An optical plethysmograph can also be used as a sensor 4 to assess skin color redness.

In an optical plethysmograph sensor, a light source is used with an optical detector to monitor light passing through tissue (in the case of the sensor being applied to a digit), or reflected from the tissue bed (in the case of the probe applied to the skin surface). The light source has weak absorption in tissue but strong absorption by blood, and a suitable wavelength is near infrared around 850nm. Multiple light sources at differing wavelengths with appropriate detector circuitry to register optical absorption at these wavelengths may also be used to provide additional information on arterial and venous blood saturation.

Each probe 2 may also contain a skin surface temperature sensor 4. The temperature sensor 4 may be thermocouple, resistance based, semiconductor junction etc and it monitors the local skin temperature at the probe placement site.

Each probe 2 also comprises circuitry 6 to enable transmission of measured parameters to the base unit 12. The transmission may be in analogue or digital form, via a wireless or wired connection.

In the case of wireless probes each probe 2 would also contain a power source, typically a rechargeable battery, to power the circuitry remotely from the base unit, whereas a wired probe could receive power from the base unit, for example via a USB connection.

The base unit 12 comprises: a microprocessor 14, a network adapter 19, a display 18, a memory 16 and signal processing circuitry 20.

The base unit may be battery or mains powered.

The network adapter 19 allows the communication of results via remote telemetry. This would allow, for example, remote monitoring of peripheral bypass graft patency by the surgeon using their wireless internet cellular telephone.

There may be signal processing circuitry 20 dedicated to each sensor, alternatively there may be signal processing circuitry dedicated to each probe with the signals from the different sensors of a probe being multiplexed at the input to the processing circuitry, alternatively there may be signal processing circuitry dedicated to each sensor type with the signals from the same sensors of different probes being multiplexed at the input to the processing circuitry, alternatively there may be only one signal processing circuitry with the signals from the different sensors of the different probes being multiplexed at the input to the processing circuitry.

The signal processing circuitry 20 comprises front end circuitry 21, which is used to feed a high-pass signal path and a low-pass signal path.

The front end circuitry 21 interfaces a sensor. There may be multiple front ends intended for simultaneous continuous monitoring of multiple sensors of multiple probes (as illustrated in Fig 1), or a single front end with an appropriate multiplexer switch (not illustrated).

The front end circuitry 21 for an impedance plethysmograph sensor would typically comprise a high frequency constant current generator that provides a current to the sensor and a control system for impedance detection and measurement.

The front end circuitry 21 for an optical plethysmograph sensor would typically comprise a controller for controlling the LEDs of the light source, a light sensor trans-impedance amplifier and a method of compensating for ambient light interference. This may be achieved using a time division multiplex implementation, in which there are periods when the light source is not illuminated. This allows monitoring of ambient light, which constitutes interference when the light source is illuminated. Alternatively, this may be achieved using a frequency division multiplex implementation, in which the light source is modulated and the detection system is synchronized to that modulation.

The front end circuitry 21 feeds a filtering subsystem 22, 23. This subsystem comprises a high pass filter 22 connected in parallel with a low pass filter 23. The high-pass filter 22 is the first element in the high pass signal path and the low pass filter 23 is the first element in the low pass signal path.

The output of a plethysmograph sensor typically comprises a large semi-static signal component and a small quasi-periodic time varying signal component. The semi-static component is mainly attributed to the static structures of the tissue and slowly changing venous circulation, and the small quasi-periodic signal component is attributed to the cardiac system filling the elastic arteries of the arterial blood supply which then drain.

The high pass filter allows the passage of signals with a frequency of greater than approximately 0.5Hz. It produces a differentiated quasi-periodic signal that represents arterial blood supply.

The low pass filter allows the passage of signals with a frequency of less than approximately 0.5Hz. It produces an integrated semi static signal.

The amplitude of both the semi-static the quasi periodic signal from the probe is dependant on many things, such as the anatomy of the subject (such as cutaneous fat), geometry and type of probe, efficiency of probe coupling. In the case of photo-optical plethysmography skin color and in the case of impedance plethysmography tissue water content have effects on signal magnitude. The resultant signal amplitudes cannot be directly compared between subjects, thus scaling of the arterial blood signal between subjects is desirable.

After probe placement, the front end circuitry 21 initializes by configuring itself for a mid-scale value of the semi-static signal component. In the case of impedance plethysmography, the current would be increased to provide a semi-static impedance signal mid way between acceptable limits - such as unity. A photo-optical plethysmograph would typically increase the LED intensity so the resultant semi-static signal would be mid way between a desired range, again for example unity.

The high pass fitter 22 is followed by an automatic gain system (AGS) 24 that amplifies the quasi-static signal. The AGS configures its gain so that the resultant arterial blood supply signal is mid way between a desired range, for example unity. Any blood supply increases or decreases would be accommodated within the signal ranges, reducing the likelihood of signal saturation or diminishment.

In the high pass signal path, the output of the AGS 24 is then converted from analogue form to digital form by analogue to digital converter 25. The resultant digital signal 27 is provided to the processor 14.

In the low pass signal path, the output of the low pass filter 23 is then converted from analogue form to digital form by analogue to digital converter 26. The resultant digital signal 28 is provided to the processor 14.

The ADCs may be discrete or may be contained in the microprocessor 14. The ADCs employ a sampling at least satisfying the Nyquist sampling criteria of at least twice the desired bandwidth e.g. 8 samples per second for a bandwidth of 0 to 4 Hz.

The microprocessor divides the high-pass filtered signal (the differentiated, quasi-periodic signal) 27 by the low-pass filtered signal (the integrated, semi static signal) 28 to achieve a arterial blood supply signal that is representative of arterial blood supply with little influence from the venous supply.

The microprocessor 14 then executes a program 17 stored in memory 16. The memory may be read only one-time programmable (programmed at time of manufacture) or may be upgradeable via the internet using reprogrammable FLASH memory and the appropriate network interface.

The programmed microprocessor 14 performs calculations that assess peripheral arterial bypass graft patency. The processing may occur in the frequency domain or the time domain.

The algorithm divides the arterial blood supply signal into epochs for subsequent processing. The epochs may be relatively long, for example multiple cardiac cycles in duration and may be 10 or 15 seconds.

**Frequency domain signal processing- signal strength and caliber.**

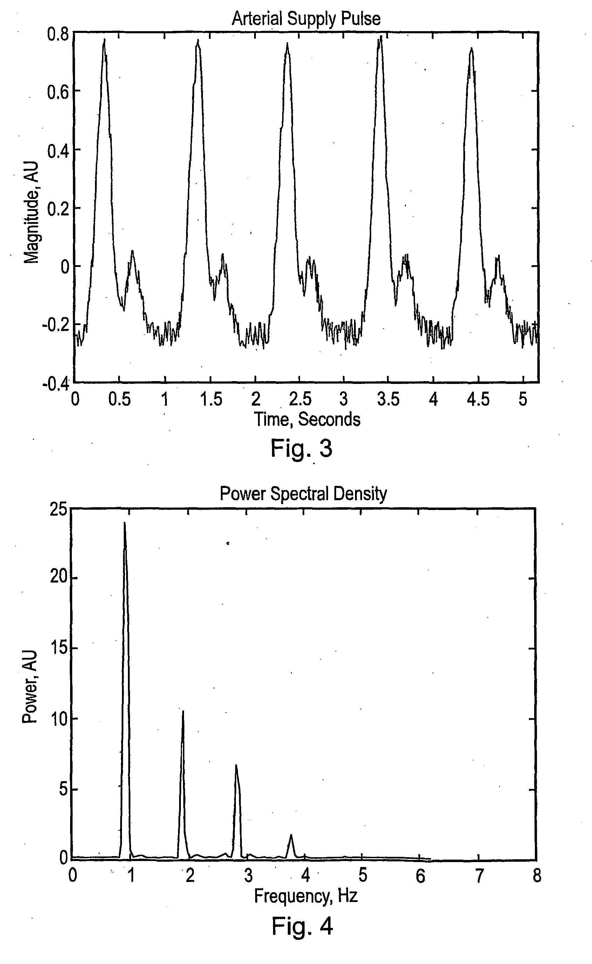

A typical arterial blood supply signal is illustrated in fig 3. Five arterial pulses are shown.

The microprocessor 14 performs frequency domain processing of the arterial blood supply signal to produce a zero-mean power spectral density (PSD) function as illustrated in Fig 4. The PSD illustrated is of a 30 second epoch from the arterial blood supply signal and is shown in fig 4 on a linear scale, 100 samples per second, a 2048 point FFT and Hamming window.

The fundamental is clearly visible in Fig. 4 with harmonics up to the fourth. The fundamental peak of a power spectral density function (PSD) is identified by the microprocessor 14.

An arterial supply signal strength is taken as the peak value in the 0 to 4Hz range since this corresponds to where the fundamental of the arterial blood supply exists - around 24 in this case.

An arterial supply signal caliber is taken as the ratio of in band to out of band components - shown here the PSD integral from 0 to 4Hz divided by the PSD integral from 4 to 8Hz.

It is important to use an epoch that is not too small as a subject may be a patient who is elderly with a degree of cardiac arrhythmia. The epoch is greater than 10 seconds, typically greater than 20 seconds. If the epochs are too short, then the signal caliber measure will fluctuate erroneously, tracking the cardiac arrhythmia. Longer epochs will produce signal quality measures over longer periods, providing greater signal strength reliability.

The frequency of the fundamental peak would also allow the determination of peripheral pulse rate (PPR), which is the frequency of cardiac pulsations in the periphery, and is closely related to heart rate.

Comparing PPR with a heart rate value derived from a central monitor, such as ECG, would provide additional information on the patency of the peripheral arterial bypass graft. If the two values were the same or similar, then there is confidence that the arterial pulsations are synchronous with the heart. If the two values are vastly different, then there is confidence that the periphery is poorly supplied by arterial blood.

**Time domain signal processing- signal strength and caliber.**

Time domain algorithms may be advantageous when using an inexpensive microprocessor 14 in the base unit 12 that is not capable of performing large Fourier transforms. Lower processing power generally implies lower current consumption, so battery power units can last longer between recharges or replacement.

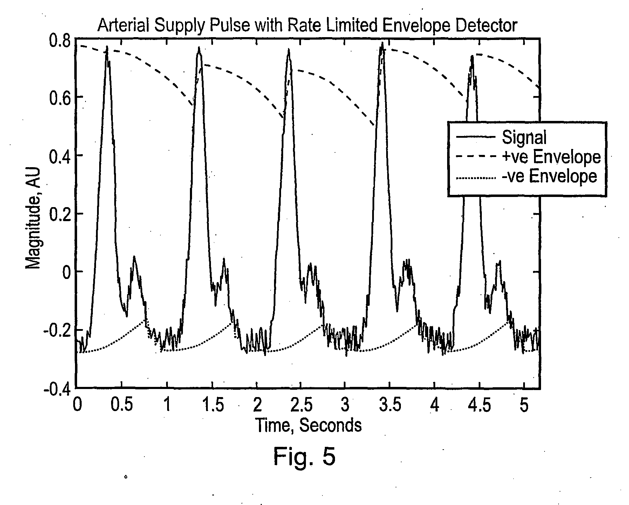

Taking the envelope of the time domain arterial blood supply signal as illustrated in Fig 5 and averaging over the epoch will provide information on arterial blood supply signal strength simply and reliably. Although a simple diode envelope detector may be used it is preferable to first use an algorithm that rejects spurious noise spikes.

The envelope of the entire epoch is then integrated to obtain a metric for the arterial blood supply strength over the epoch.

In an embodiment employing time domain analysis, arterial blood supply caliber may be determined using correlation techniques.

The size of the largest non-zero peak of an auto-correlation function is a suitable arterial blood supply caliber metric. However, this is an absolute value and is not comparable between subjects.

An improved implementation is the comparison by cross correlation of the signal for the current epoch with the signal for a preceding epoch. The preceding epoch may be the first epoch following initialization, for example, or alternatively the immediately preceding epoch.

The two epochs are stored in single dimension data arrays (vectors) of three times the length of each epoch, in the microprocessor memory. One epoch is placed at the start of the array (stored), the other epoch is placed in the centre of the array (current). The remaining array locations are filled with zeros.

The Pearson Correlation coefficient for the two arrays is obtained by straightforward calculation, and also placed in a result array. The stored array values are then shifted one sample point along, filling the empty portion of the array with zeros. The Pearson correlation coefficient is then calculated again between the shifted stored epoch and the current epoch in the centre of the array.

This continues until the stored epoch has moved across the entire current epoch, producing an array of Pearson correlation results that oscillates between positive and negative values within unity. The maximum value of the Pearson Correlation array can be used as the arterial blood supply caliber metric, and in addition, the spacing in samples between the maximum and minimum values of the Pearson Correlation array can be used to calculate average peripheral pulse rate for the current epoch. Advantages of this technique are that the arterial metric will always range between plus and minus unity.

**Skin Color Redness**

An embodiment using photo-optical plethysmography may also optionally monitor skin color redness for each probe location. Monitoring of skin color redness can provide information on venous blood color, which will be influenced by the arterial blood supply and venous drainage in the region of probe location. Skin color redness (SCR) can be determined using a multi wavelength photo-electric probe, with wavelengths sensitive to oxy Hb and deoxy Hb, typically 640nm (Red) and 840nm (IR). A skin color redness indicator may be calculated for each probe using a function of the semi-static signals 28 from the Red sensor and the IR sensor, for example,

$$SCR = 2 * Semi\text{-}Static\_Red / ( Semi\text{-}Static\_Red + Semi\text{-}Static\_IR )$$

In this example, upon initialization the SCR value would be unity, since the Semi-static signal 28 and quasi-periodic signal 27 are initialized to unity using AGS 24. Deviations in skin color redness could be greater than unity to indicate increased venous oxygen saturation, or less than zero to indicate decreased oxygen saturation.

**Skin temperature**

Skin temperature at each probe location may also, optionally, be monitored. Monitoring of skin temperature relative to ambient temperature can provide information on blood perfusion, which will be influenced by the arterial blood supply in the region of probe location.

For example the rate of change in the difference between skin temperature and ambient temperature over time may be monitored at each probe using the semi-static signals 28 from the temperature sensor. A reduction in the response time of the skin to an ambient temperature variation or a reduction in the difference between skin temperature and ambient temperature may indicate a reduction in arterial blood supply.

**Processing**

After the microprocessor 14 has calculated the various arterial blood supply metrics which include a plurality of: arterial blood supply strength, arterial blood supply caliber, skin color redness, skin temperature, and peripheral pulse rate, they may be displayed individually on the display 18 of the base unit 12.

The microprocessor 14 uses a predefined function that takes as arguments several of these arterial blood supply metrics to determine a combined arterial patency metric. The function can scale or use non linear processing to weight the various input arterial blood metrics.

Let there be arterial blood supply metrics m1, m2 ... mN and the arterial blood supply metrics be combined, using the predetermined function F, to create the combined arterial patency metric P , where P = F(m1, m2, ...mN)

If one treats the arterial blood supply metrics as independent metrics (which is not necessarily true), then $dF/\Pi \, dmi = g(mj)$ where i=1, 2 ... N but i ≠ j. The solution to this equation is F=$\Pi$ mi where i=1, 2..N.

For example, using the values stated previously, the arterial blood supply strength may be multiplied by the arterial blood supply signal caliber, multiplied by the skin color redness to generate a patency indicator. Since a low value in any of these components indicates reduced graft patency, the combined metric would be sensitive to a fall in any single parameter.

It is preferable to use values of the metrics that have been normalized to 1. This may be achieved by dividing the metric value at the current time with the metric value at initialization.

Comparing the combined arterial patency metrics amongst, say three probe locations on the foot, will indicate when a single artery such as peroneal, is loosing patency. The microprocessor 14 calculates a difference between the combined arterial patency metric for a first arterial blood supply at a first tissue area and the combined arterial patency metric for at least a second arterial blood supply at a second tissue area.

If an artery is not patent, the combined arterial patency metric for the probe location corresponding to that artery, will decrease relative to the other probe locations for other arteries. Thus the relative value of a combined arterial patency metric at a first location compared to the combined arterial patency metric at a second location at the same time may be used to assess the patency of the artery feeding the first location.

The first and second locations correspond to first and second arteries. The first and second arteries may be parallel branches of another artery, such as the arteries of the foot (first and second locations). The first and second arteries may be serial arteries, where the first artery is an artery in the foot (first location) that has branched from the second artery which passes behind he knee (second location).

The rate of change of a combined arterial patency metric may be used to provide similar information. Thus the relative value of a combined arterial patency metric at a first location compared to the combined arterial patency metric at a same location at a previous time may be used to assess the patency of the artery feeding the first location

The microprocessor 14 could be configured so an alarm is activated on the display 18 or via the network adapter 19 to indicate when a metric has exceeded a certain threshold. This may be upper or lower thresholds, and may be any of the parameters monitored, or the arterial blood supply metrics or the combined arterial patency metric.

An embodiment employing an internet connection could send an email to the clinician indicating the time and date of the alarm, so the clinician would be alerted when using their mobile internet cellular phone to check their email. An extension of this concept is to send an email or mobile telephone message (e.g. SMS, MMS) thus allowing a message to be sent to the clinician, alerting them to the situation.

**Probe Positioning Apparatus**

Fig. 2 illustrates an example of a probe positioning apparatus 40. The apparatus 40 may be may be a holster, sandal or sock arrangement to which the probes 2 are secured or removably securable in a number of predefined locations, the figures illustrates a holster. Each predefined location overlies a tissue bed predominantly fed by a specific artery when the apparatus is in use. Example arteries are: anterior tibial artery which becomes the dorsalis pedis artery, posterior tibial artery, plantar artery, and peroneal artery.

A suitable location for assessing the blood supply from the dorsalis pedis artery is between the extensor hallusis longus and the second metatarsal, usually towards the instep of the dorsum of the foot. The probe 2B is placed at this location in Fig 2.

A suitable location for assessing the blood supply from the posterior tibial artery is between the medial malleolus and the medial tuberosity of the calcaneus (inside of the foot beneath the ankle). The probe 2A is placed at this location in Fig 2.

A suitable location for sampling the blood supply from the plantar artery is the area near the base of the toes.

A suitable location for sampling the blood supply from the peroneal artery is on the lateral aspect of the foot between the lateral malleolus and calcaneus (heel). The probe 2C is placed at this location in Fig 2.

The apparatus 40 typically comprises a substrate 42 that contacts a subject's foot and that has predetermined locations 44 at which probes 4 may be attached. The substrate 42 is held in place against the user's foot by, for example, integrating the substrate into a sock, forming the substrate as a sandal or holster, using glue to attach the substrate or strapping the substrate in place. The substrate 42 is preferably disposable being discarded after single use after removal of some or all of the probes 4, where use, for example, constitutes monitoring peripheral arterial blood supply for several hours after bypass surgery.

It is important to prevent the substrate rotating about the lower end of the calf. In the examples illustrated, this is achieved by having the substrate straddle, surround or enclose the maleoulus.

In Fig. 2 the apparatus 40 is a holster. The design may be used on the opposite foot as the holster is roughly adjustable. A matrix probe (described below) is suitable for use with the design of Fig 2 as it allows general as opposed to accurate placement of a probe. The design does not require any location by toes anatomy, which is an important consideration in the patient subgroup undergoing peripheral arterial bypass, as they may have already had toes removed.

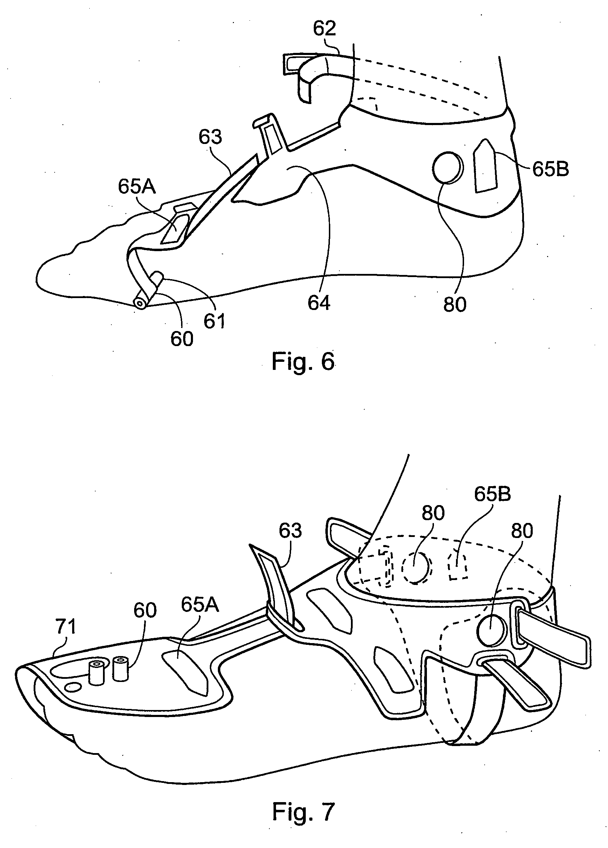

Alternative holster designs are illustrated in Figs 6, 7 and 8. In one embodiment, illustrated in Fig 6, the substrate 42 of holster 40 is positioned and oriented by the use of a 'toe brace' 60 which passes between the great toe and its neighboring toe and by use of cut-outs 80 which reference the maleolus. The cut-outs 80 prevent rotation of the substrate 42 about the talus. The toe brace has a flange 61 that is located on the underside of the foot between the great toe and its neighbor. The toe brace 61 is connected to the substrate 42 which extends over the metatarsals and dorsum and then around the ankle in a loop 62. A tensioning mechanism 63 is provided between the toe brace and the ankle loop 62. In the example illustrated the substrate can be pulled taught and stuck down using 'one-use-only' adhesive on the substrate. The ankle loop 62 may also be secured using similar adhesive. The substrate has apertures 65A and 65B for releasably receiving probes 2A and 2B respectively. The aperture 65A is located over the plantar artery and the aperture 65B is located over the posterior tibial artery. A further aperture for a sensor could be provided on the substrate over the dorsum 64 of the foot for sampling the dorsalis pedis artery. The substrate may be provided as a reversible (invertible) flat die which is used with one face uppermost on the left foot and another face uppermost on the right foot. In this embodiment, there is a heel cut-out and the toes of the patient are not enclosed as in a sock. The substrate material is preferably slightly resiliently deformable and the apertures 63 are sized for a snug fit with the housing of the respective probes 2. A barrier membrane may be provided in each aperture.

Combined with the big toe brace 61, the cut-outs 80 and the adjustment tab 63 combine to prevent movement of the substrate 42 and the probes 2 in use. The design still allows conventional access to the foot for traditional assessment such as skin temperature or palpation assessment by nurse, which would not be possible with an enclosing sock design. In addition, the adjustment tab 63 and 62 allows a single design to fit many feet sizes with reversibility (invertibility) making the design suitable for both left and right feet. In addition, this design does not rely on toes being a certain shape - elderly people can have hooked or clawed toes very different to normal toes, and this design only requires the presence of two toes adjacent.

In another embodiment, illustrated in Fig 7, the substrate 42 of holster 40 is positioned and oriented by the use of a 'toe brace' 60 which passes between the great toe and its neighboring toe. The toe brace is connected to the substrate 42 which forms a wrap 71 around the exterior instep side only of the great toe. The substrate 42 extends over the metatarsals and dorsum and then around the ankle in a loop 62. A tensioning mechanism 63 is provided between the toe brace and the ankle loop 62. In the example illustrated the substrate can be pulled taught and stuck down using 'one-use-only' adhesive on the substrate. The ankle loop 62 may also be secured using similar adhesive. The substrate has apertures 65A and 65B for releasably receiving probes 2A and 2B respectively. The aperture 65A is located over the plantar artery and the aperture 65B is located over the posterior tibial artery. A further aperture for a sensor could be provided on the substrate over the dorsum of the foot for sampling the dorsalis pedis artery. The substrate 42 may be provided as a two-part reversible (invertible) flat die which is used with one face uppermost on the left foot and another face uppermost on the right foot. In this embodiment, there is a heel cut-out and the toes of the patient are not enclosed as in a sock. The substrate material is preferably slightly resiliently deformable and the apertures 63 are sized for a snug fir with the housing of the respective probes 2. A barrier membrane may be provided in each aperture.

The assembly is prevented from rotating about the talus by the maleolus locating cut-out holes 80. Apertures can be provided for the posterior tibial (inside foot), peroneal, dorsalis pedis, plantar and, due to the toe enclosure, access to the metatarsal arteries of the great toes. For optical techniques, this can be reflection or transmission using a sensor diametrically opposite. This design still allows conventional access to the toes for traditional assessment such as skin temperature or palpation assessment by a nurse, which would not be possible with an enclosing sock design

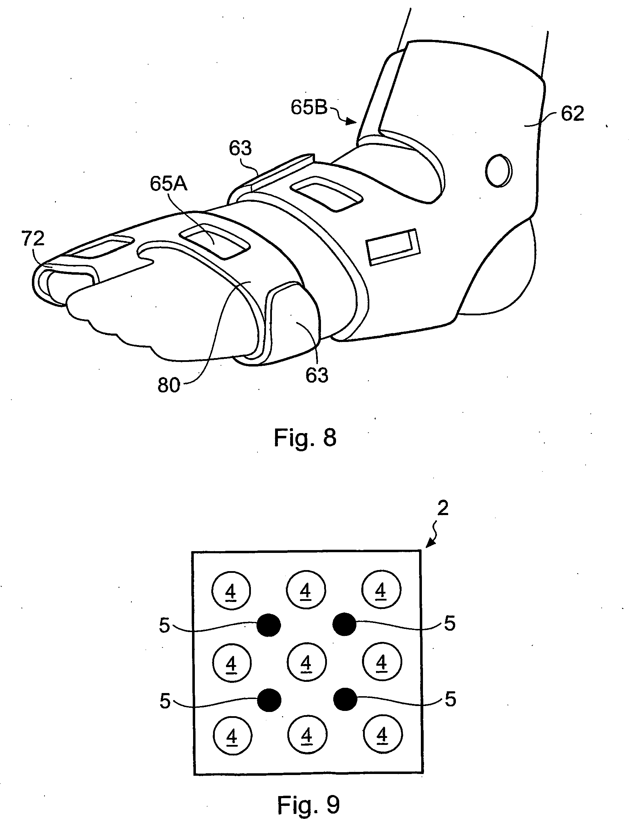

In another embodiment, illustrated in Fig 8, the substrate 42 is a two-part substrate. A first part 80 is positioned and oriented by the use of a 'great toe enclosure' 72 which passes between the great toe and its neighboring toe and is wrapped around the exterior of the great toe. The substrate 42 extends over the metatarsals and forms a loop under the sole of the foot. A second part 80 is positioned and oriented by the use of an ankle loop and a loop around the dorsum and sole of the foot. A tensioning mechanism 63 is provided in each of the ankle and sole loops using 'one-use-only' adhesive on the substrate. The substrate has apertures 65A and 65B for releasably receiving probes 2A and 2B respectively. The aperture 65A is located over the plantar artery and the aperture 65B is located over the posterior tibial artery. A further aperture for a sensor could be provided on the substrate over the dorsum of the foot for sampling the dorsalis pedis artery. Each part substrate of the substrate may be provided as a foam reversible (invertible) flat die which is used with one face uppermost on the left foot and another face uppermost on the right foot. In this embodiment, there is a heel cut-out and the toes of the patient are not enclosed as in a sock. The substrate material is preferably slightly resiliently deformable and the apertures 63 are sized for a snug fit with the housing of the respective probes 2. A barrier membrane may be provided in each aperture.

This design may be developed from a porous disposable sponge allowing the skin to breathe for the long periods the holster may be worn. The features are essentially the same as fig 7 but using different material and number of parts. The foam may be used to mount the probes using the natural elasticity of the material, using a shaped hole. The foam also acts as a semi rigid substrate to help reduce probe movement problems (particularly appropriate for the toe sensor in this design), and also acts as a light barrier, and again may be reversible (invertible). This design still allows conventional access to the toes for traditional assessment such as skin temperature or palpation assessment by nurse, which would not be possible with an enclosing sock design

The location of the probes 2 is especially important when below-the-knee bypass grafts are performed, since individual arterial branches thus arterial blood supply routes may be monitored using multiple probes on the foot, monitoring specific arteries of the foot.

As will be, appreciated from the foregoing, it is important that an arterial sensor is accurately positioned relative to the subject artery. This may be achieved by the accurate and stable positioning of the probe carrying the sensor or by an automated process as described below.

Fig 9 illustrates a probe 2 comprising an arrangement of identical optical sensors 4 and corresponding light sources 5. In the example, the sensors 4 are arranged as a 3x3 grid array and the light sources 5 are arranged as a 2x2 grid array off-set from the 3x3 array. The columns of sensors are separated from adjacent columns of sensors by 5mm to 10mm and the rows of sensors are separated from adjacent rows of sensors by 5mm to 10mm. The rows and columns of light sources 5 are similarly separated.

The probe 2 has many times the sensing area of a single sensor and light source pair. During the initialization phase of the base unit 12, the base unit 12 scans the sensors 4 to identify the sensor 4 with the strongest arterial supply signal strength. It uses this identified sensor 4 and disregards the remaining sensors of that particular probe for the rest of the observation session.

The base unit 12 determines for each sensor of the multi-sensor probe 2, a potential arterial blood supply signal and determines a 'best' potential arterial blood supply signal or signals and consequently a best sensor or sensors. It then uses the output of the best sensor or sensors only for further processing.

As described previously, the output of each sensor 4 is filtered through a low-pass filter 23 and a high-pass filter 22 in parallel. The microprocessor 14 divides the high-pass filtered signal (the differentiated, quasi-periodic signal) 27 by the low-pass filtered signal (the integrated, semi static signal) 28 to achieve a potential arterial blood supply signal that may be representative of arterial blood supply.

The 'best' signal may be selected from any one of or any combination of: the amplitude of the high-pass filtered signal, the amplitude of the potential arterial blood supply signal or the amplitude of the power spectral density (PSD) of the potential arterial blood supply signal.

The use of only the output from the best sensor or sensors may be achieved either by instructing the probe 2 to operate only the best sensor or sensors 4 or by processing the inputs from all the sensors 4 of the probe 2 to select only the output(s) from the best sensor(s) for further processing i.e. calculating of an arterial blood supply signal.

Although the base unit 12 has been described as a separate perhaps distant unit to the probes 2, it may be integrated into the apparatus as illustrated in Fig 2, where it is mounted to the dorsum of the foot.

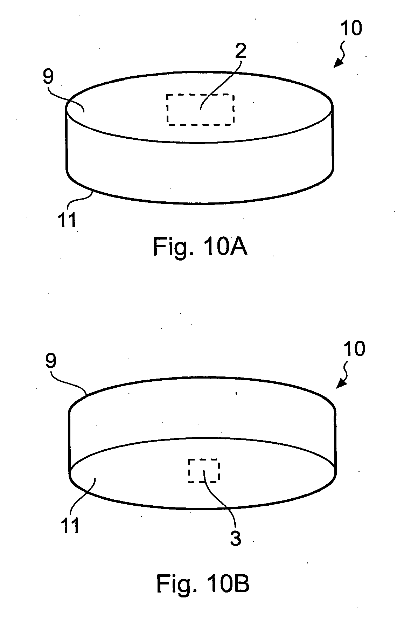

The base unit may also be integrated into a substrate as illustrated in Fig 10 to form the compact PAPM system 10... In Fig 10, the base station and a probe 2A are integrated within a substrate 7 for attachment to a limb of the subject using, for example, stick-on hydro-gel, sticky tape, bandages or a bracelet. The substrate forms a small (<16 cm$^2$), light-weight, self-contained, low-cost PAPM system 10 that measures perfusion at a single location using one or possibly more sensors. The PAPM system 10 may be disposable. A visual indicator, such as an LED may be used to demonstrate arterial patency or a change in arterial patency.

The PAPM system 10 comprises a probe 2, processing circuitry 20 as described in relation to Fig 2. It also has a visual indicator 3 but does not have the network interface 19 and display 18. Depending upon implementation it may, or may not, have the microprocessor 14 and memory 16.

In a compact 'pill' embodiment, illustrated in Fig 10, the PAPM system 10 is shaped as a cylindrical tube that is 25mm in diameter and 15mm tall. A probe 2 with one or more sensors is positioned on a first flat face 9 and one or more visual indicators 3 are positioned on the other second flat face. The first flat face 9 of the compact monitor 10 would affix to a fleshy, well perfused region of the patient's skin, such as calf or foot.

In one embodiment, a light source of the probe 2 in the first face 9 illuminates the pulsatile, arterial blood vessels in the dermis and in sub-cutaneous tissues adjacent the first face 9 and one or more light sensors of the probe 2 in the first flat face 9 detect the light reflected from the arterial blood vessels.

A wavelength of light is used which is not strongly absorbed by tissue, is strongly absorbed by whole blood but with an absorption that varies little with hemoglobin oxygen saturation, such as 810nm. The modulation of the reflected light by the illuminated arterial bed is synchronous to the change in arterial diameter caused by the cardiovascular pulse wave, and detectible with the light sensor and subsequent filtering and amplification by the processing circuitry 20 as described previously in relation to Fig 1.

The determined peripheral pulse rate may be used to drive the visual indicator 3 on the second side 11 of the compact monitor 10. The visual indicator may be a LED which is controlled so that is flashing frequency corresponds to the PPR.

The color of the visual indicator 3 (or number of flashing indicators) may be varied in dependence upon the determined arterial blood supply strength or similar metric. A simple traffic light approach could be implemented, using a signal strength algorithm, dividing the range of perfusion strength into three zones:

1. high perfusion strength as Green

2. medium perfusion strength as Yellow

3. low perfusion strength as Red

A combined arterial patency metric may be created by multiplying arterial blood supply metrics together such as a normalized value for the PPR and a normalized value of the arterial blood supply strength or caliber. The combined arterial patency metric may then be used to control the traffic light color of the visual indicator 3 (or the number of LEND indicators used). A piezoelectric beeper may be sounded if the combined arterial patency metric falls below a threshold.

If multiple light sources were employed, then skin color redness may also be introduced as an arterial blood supply metric. Here, a light source illuminates the non-pulsatile capillaries in the epidermis. The perfusion strength and skin color metric may then be combined using a predetermined function, as described previously, to generate a combined patency metric.

Skin temperature may be used as a further arterial blood supply metric which is combined to create the patency metric.

The arterial blood supply metrics are normalized by taking and recording initial values when first affixing the device to the skin and then dividing subsequent values by the initial values. This compensates for inherent variations in skin thickness, skin tone and vascular systems.

A light source in the probe provides short pulses of illumination. To assess arterial pulse waves with low power consumption, the light pulses would be typically 50us wide. The light sensor would, assuming maximum peripheral pulse rate of 3.5 heart beats per second, be sampled at 10 times per second. To assess skin color redness and/or skin temperature, then the light pulses and subsequent sampling may be less frequent, such as once per second.

To increase the sensitivity of the device, a light sensor and light source in the probe 2 could be designed with optical diffusers. The diffusers effectively increase the active area of the probe 2, reducing sensitivity to location and alignment.

A consumable implementation of the invention could employ a Zinc Air battery as a power source. Zinc air batteries have the advantage of very low self discharge rates when not activated.

An alternative embodiment would employ a lithium micro power cell as a power source. These batteries have long life but low energy densities, and are not rechargeable. This approach may need a transient current buffer system as small lithium batteries have high internal impedance and cannot deliver high current pulses, as is required for light source flashes.

A reusable self-contained monitor 10 would have a rechargeable battery. The compact monitor 10 could contain a wire coil in the outer housing to act as a wireless energy transfer device in order to charge the battery. The coil could also act as a quiescent energy store to power high power current transients required by the light source and also to flash the visual indicator light sources.

A multitude of thermocouples may be integrated into the first side 9 of the monitor 10 so that they are in thermal contact with the patient's skin to form a thermopile power source. The thermocouple would also provide a skin temperature reference, which could be used as an arterial blood supply metric and combined with other blood supply metrics to form an arterial patency metric. Such a micro power source requires a means of coping with the transient power requirements of the device. An inductor or capacitor would be energized using the micro power energy, and a state machine timing system controls discharge of the stored energy through the light sensor, and the visual indicator at the required time.

It may be necessary to modify the parameters of the device, such as the alarm thresholds. As the compact monitor 10 already contains a light sensor and light source in the probe 2, these could be reused to provide an infra red data link to a computer running a configuration utility. A reconfigurable microprocessor 14 with integrated analogue and digital subsystem could perform the architecture reshuffle on the fly. Equipping the device with a memory 16 and clock would allow time stamping of particular events and intervals, allowing interrogation of stored metrics. For example, periods where the arterial blood supply strength fell below a defined value during the night could be logged as events to memory, then interrogated and downloaded in the morning.

Although embodiments of the present invention have been described in the preceding paragraphs with reference to various examples, it should be appreciated that modifications to the examples given can be made without departing from the scope of the invention as claimed.

**Claims**

1.  A post-operative, arterial patency monitoring system for monitoring patency of an artery of a limb comprising:

    a first probe adapted to be positioned adjacent a first tissue area of a limb for producing first output signals dependent upon a first arterial blood supply to the first tissue area;
    a second probe adapted to be positioned adjacent a second different tissue area of the limb for producing second, different output signals dependent upon a second arterial blood supply to the second tissue area; and
    means for processing the first output signals to produce a first arterial blood supply metric for the first arterial blood supply and a second, different, arterial blood supply metric for the first arterial blood supply;
    means for processing the second output signals to produce the first arterial blood supply metric for the second arterial blood supply and the second arterial blood supply metric for the second arterial blood supply;
    means for using a predetermined function that takes as its arguments at least the first and second arterial blood supply metrics for the first arterial blood supply to produce a combined arterial patency metric for the first blood supply;
    means for using the predetermined function that takes as its arguments at least the first and second arterial blood supply metrics for the second arterial blood supply to produce a combined arterial patency metric for the second arterial blood supply;
    means for comparing the combined arterial patency metric for the first arterial blood supply with the combined arterial patency metric for the second arterial blood supply; and
    means for determining the patency of a first artery that provides the first arterial blood supply using a difference between the combined arterial patency metric for the first arterial blood supply and the combined arterial patency metric for the second arterial blood supply.

2.  A system as claimed in claim 1, wherein the predetermined function multiplies together at least the first and second arterial blood supply metrics to producer a combined arterial patency metric and wherein the first arterial blood supply metric and the second arterial blood supply metric are normalized before multiplication.

3.  A system as claimed in any preceding claim, wherein the first arterial blood supply metric and the second arterial blood supply metric are different ones of: arterial blood supply signal strength, arterial blood supply signal caliber, peripheral pulse rate, skin color redness and skin temperature.

**4.** A system as claimed in any preceding claim, wherein each probe comprises at least a first sensor and a second sensor, wherein the first sensor provides first output signals used to produce the first arterial blood supply metric and wherein the second sensor provides first output signals used to produce the second arterial blood supply metric and wherein the first and second sensors are different ones of: a plethysmograph sensor, a skin temperature sensor and a skin color sensor.

**5.** A system as claimed in any preceding claim further comprising signal processing circuitry for processing the output of a sensor into a high frequency signal and a low frequency signal and further comprising means for forming a ratio of the high frequency signal to the low frequency signal.

**6.** A system as claimed in any preceding claim, wherein at least one of the first and second probes has a plurality of sensors, the system further comprising means for processing the outputs from the plurality of sensors to select a subset of the plurality of sensors in dependence upon ac components of the outputs from the plurality of sensors and operable subsequently to process the outputs of only the selected subset of sensors to assess blood flow.

**7.** A system as claimed in any preceding claim, further comprising a probe positioning apparatus comprising:

a three dimensional substrate shaped to conform to a foot;
means for correctly orientating and positioning the substrate adjacent a predetermined portion of a foot when the apparatus is in use;
a first probe location means at a predetermined first location on the substrate such that when the apparatus is in use, the first location is adjacent the first tissue are and the first artery, and
a second probe location means at a predetermined second location on the substrate such that when the apparatus is in use, the second location is adjacent the second tissue area and a second artery.

**8.** A system as claimed in claim 7, wherein
the three dimensional substrate is invertible such that in a first configuration the substrate is shaped to conform to a left foot and in a second configuration, which is inverted in comparison to the first configuration, the substrate is shaped to conform to a right foot, wherein the means for correctly orientating and positioning the substrate is operable in the first configuration and the second configuration, wherein the first location of the first probe location means is such that when the probe positioning apparatus is in use, the first location is adjacent the same first tissue area of the foot irrespective of whether it is used in the first configuration with the left foot or in the second configuration with the right foot and wherein the second location of the second probe location means is such that when the probe positioning apparatus is in use, the second location is adjacent the same second tissue area of the foot irrespective of whether it is used in the first configuration with the left foot or in the second configuration with the right foot.

**9.** A system as claimed in claim 7 or 8, wherein the first and second arteries are selected from the group comprising: dorsalis pedis, posterior tibial, plantar and peroneal of the same foot.

**10.** A system as claimed in claim 7, 8 or 9, wherein the first and second tissue areas are selected from the group comprising: the tibial side of the dorsum of the foot, the inside of the foot beneath the ankle, between the first and second metatarsal bones, the great toe and the heel of the foot.

**11.** A system as claimed in any preceding claim, wherein a second artery provides the second arterial blood supply and the first and second arteries are parallel branch arteries from a third artery or wherein a second artery provides the second arterial blood supply and the first artery is a downstream branch of the second artery.

**12.** A system as claimed in claim 8, wherein the first tissue area is part of a foot and the second tissue area is behind a knee of the same leg.

**13.** A signal processing unit of a post-operative, arterial patency monitoring system for monitoring patency of an artery of a limb, the unit comprising:

a first input for receiving first input signals from a first probe that are dependent upon a first arterial blood supply to a first tissue area of a limb;
means for processing the first output signals to produce a first arterial blood supply metric for the first arterial blood supply and a second, different, arterial blood supply metric for the first arterial blood supply;
means for using a predetermined function that takes as its arguments at least the first and second arterial blood supply metrics for the first arterial blood supply to produce a combined arterial patency metric for the first arterial blood, supply;

a second input for receiving second input signals from a second probe that are dependent upon a second arterial blood supply to a second tissue area of the limb;

means for processing the second output signals to produce a first arterial blood supply metric for the second arterial blood supply and a second, different, arterial blood supply metric for the second arterial blood supply;

means for using the predetermined function that takes as its arguments at least the first and second arterial blood supply metrics for the second arterial blood supply to produce a combined arterial patency metric for the second arterial blood supply;

means for comparing the combined arterial patency metric for the first arterial blood supply with the combined arterial patency metric for the second arterial blood supply; and

means for determining the patency of a first artery that provides the first arterial blood supply using a difference between the combined arterial patency metric for the first arterial blood supply and the combined arterial patency metric for the second arterial blood supply.

14. A unit as claimed in claim 13 further comprising signal processing circuitry for processing the output of a sensor into a high frequency signal and a low frequency signal.

15. A post-operative, arterial patency monitoring method for monitoring patency of an artery of a limb comprising:

positioning a first probe adjacent a first tissue area of a limb fed by a first arterial blood supply such that it produces first output signals that are dependent upon the first arterial blood supply to the first tissue area;

positioning a second probe adjacent a second tissue area of the limb fed by a second arterial blood supply such that it produces second output signals that are dependent upon the second arterial blood supply to the first tissue area;

processing the first output signals to produce a first arterial blood supply metric for the first arterial blood supply and a second, different, arterial blood supply metric for the first arterial blood supply;

combining at least the first and second arterial blood supply metrics for the first arterial blood supply, using a predetermined function that takes as its arguments at least the first and second arterial blood supply metrics for the first arterial blood supply, to produce a combined arterial patency metric for the first blood supply;

processing the second output signals to produce a first arterial blood supply metric for the second arterial blood supply and a second, different, arterial blood supply metric for the second arterial blood supply;

combining at least the first and second arterial blood supply metrics for the second arterial blood supply, using the predetermined function that takes as its arguments at least the first and second arterial blood supply metrics for the second arterial blood supply, to produce a combined arterial patency metric for the second arterial blood supply;

comparing the combined arterial patency metric for the first arterial blood supply with the combined arterial patency metric for the second arterial blood supply; and

determining the patency of a first artery that provides the first arterial blood supply using a difference between the combined arterial patency metric for the first arterial blood supply and the combined arterial patency metric for the second arterial blood supply.

## Patentansprüche

1. Überwachungssystem für post-operative Arterien-Durchgängigkeit zum Überwachen der Durchgängigkeit einer Arterie eines Gliedes, umfassend:

eine erste Sonde, die geeignet ist, um neben einem ersten Gewebebereich eines Glieds positioniert zu werden, um erste Ausgabesignale zu erzeugen, die von einer ersten arteriellen Blutzufuhr zu dem ersten Gewebebereich abhängig sind;

eine zweite Sonde, die geeignet ist, um neben einem zweiten, unterschiedlichen Gewebebereich des Glieds positioniert zu werden, um zweite, unterschiedliche Ausgabesignale zu erzeugen, die von einer zweiten arteriellen Blutzufuhr zu dem zweiten Gewebebereich abhängig sind; und

Mittel zum Verarbeiten der ersten Ausgabesignale, um eine erste arterielle Blutzufuhr-Metrik für die erste arterielle Blutzufuhr und eine zweite, unterschiedliche arterielle Blutzufuhr-Metrik für die erste arterielle Blutzufuhr zu erzeugen;

Mittel zum Verarbeiten der zweiten Ausgabesignale, um die erste arterielle Blutzufuhr-Metrik für die zweite arterielle Blutzufuhr und die zweite arterielle Blutzufuhr-Metrik für die zweite arterielle Blutzufuhr zu erzeugen; Mittel zum Verwenden einer vorbestimmten Funktion, die als ihre Argumente zumindest die erste und die zweite arterielle Blutzufuhr-Metrik für die erste arterielle Blutzufuhr nimmt, um eine kombinierte arterielle Durchgängigkeit-Metrik für die erste Blutzufuhr bereitzustellen; Mittel zum Verwenden der vorbestimmten Funktion, die als ihre Argumente zumindest die erste und die zweite arterielle Blutzufuhr-Metrik für die zweite arterielle Blutzufuhr nimmt, um eine kombinierte arterielle Durchgängigkeit-Metrik für die zweite arterielle Blutzufuhr bereitzustellen; Mittel zum Vergleichen der kombinierten arteriellen Durchgängigkeit-Metrik für die erste arterielle Blutzufuhr mit der kombinierten arteriellen Durchgängigkeit-Metrik für die zweite arterielle Blutzufuhr; und Mittel zum Bestimmen der Durchgängigkeit einer ersten Arterie, welche die erste arterielle Blutzufuhr bereitstellt, unter Verwendung einer Differenz zwischen der kombinierten arteriellen Durchgängigkeit-Metrik für die erste arterielle Blutzufuhr und der kombinierten arteriellen Durchgängigkeit-Metrik für die zweite arterielle Blutzufuhr.

2. System nach Anspruch 1, bei welchem die vorbestimmte Funktion zumindest die erste und die zweite arterielle Blutzufuhr-Metriken miteinander multipliziert, um eine kombinierte arterielle Durchgängigkeit-Metrik zu erzeugen, und wobei die erste arterielle Blutzufuhr-Metrik und die zweite arterielle Blutzufuhr-Metrik vor der Multiplikation normiert werden.

3. System nach einem der vorhergehenden Ansprüche, bei welchem die erste arterielle Blutzufuhr-Metrik und die zweite arterielle Blutzufuhr-Metrik unterschiedlich sind und aus der folgenden Gruppe stammen: Signalstärke der arteriellen Blutzufuhr, Signalkaliber der arteriellen Blutzufuhr, periphere Pulsgeschwindigkeit, Hautfarben-Röte und Haut-Temperatur.

4. System nach einem der vorhergehenden Ansprüche, bei welchem jede Sonde zumindest einen ersten Sensor und einen zweiten Sensor aufweist, wobei der erste Sensor erste Ausgabesignale bereitstellt, die zur Erzeugung der ersten arteriellen Blutzufuhr-Metrik verwendet werden, und wobei der zweite Sensor erste Ausgabesignale bereitstellt, die zur Erzeugung der zweiten arteriellen Blutzufuhr-Metrik verwendet werden, und wobei der erste und der zweite Sensor unterschiedlich sind und aus der folgenden Gruppe stammen: Plethysmograph-Sensor, Hauttemperatur-Sensor und Hautfarben-Sensor.

5. System nach einem der vorhergehenden Ansprüche, welches ausserdem aufweist: eine Signalverarbeitungs-Schaltung zum Verarbeiten der Ausgabe eines Sensors zu einem Hochfrequenz-Signal und einem Niederfrequenz-Signal, sowie ein Mittel zum Bilden eines Verhältnisses des Hochfrequenz-Signals zu dem Niederfrequenz-Signal.

6. System nach einem der vorhergehenden Ansprüche, bei welchem zumindest die erste und/oder die zweite Sonde eine Vielzahl von Sensoren hat, wobei das System ausserdem Mittel zum Verarbeiten der Ausgaben von der Vielzahl der Sensoren hat, um eine Teilmenge der Vielzahl von Sensoren in Abhängigkeit von Wechselsignal-Komponenten (ac-Komponenten) der Ausgaben von der Vielzahl der Sensoren auszuwählen, und anschliessend betätigbar ist, um die Ausgaben nur der ausgewählten Untergruppe der Sensoren zu verarbeiten, um die Blutströmung zu beurteilen.

7. System nach einem der vorhergehenden Ansprüche, welches ausserdem eine Sonden-Positioniervorrichtung aufweist, umfassend:

eine dreidimensionale Unterlage, die zur Anpassung an einen Fuss geformt ist; Mittel zum korrekten Orientieren und Positionieren der Unterlage neben einem vorbestimmten Abschnitt eines Fusses, wenn die Vorrichtung in Betrieb ist; ein erstes Sondenort-Mittel an einem vorbestimmten ersten Ort auf dem Substrat, so dass, wenn die Vorrichtung in Betrieb ist, der erste Ort neben dem ersten Gewebebereich und der ersten Arterie ist; und ein zweites Sondenort-Mittel an einem vorbestimmten zweiten Ort auf dem Substrat, so dass, wenn die Vorrichtung in Betrieb ist, der zweite Ort neben dem zweiten Gewebebereich und einer zweiten Arterie ist.

8. System nach Anspruch 7, bei welchem die dreidimensionale Unterlage umkehrbar ist, so dass in einer ersten Konfiguration die Unterlage zur Anpassung an einen linken Fuss geformt ist und in einer zweiten Konfiguration, die im Vergleich zur ersten Konfiguration umgekehrt ist, die Unterlage zur Anpassung an einen rechten Fuss geformt ist, wobei das Mittel zum korrekten Orientieren und Positionieren der Unterlage in der ersten Konfiguration und der zweiten Konfiguration betätigbar ist, wobei der erste Ort des ersten Sondenort-Mittels derart ist, dass beim Betrieb der Sonden-Positioniervorrichtung der erste Ort neben demselben ersten Gewebebereich des Fusses ist, unabhängig davon, ob sie in der ersten Konfiguration mit dem linken Fuss oder in der zweiten Konfiguration mit dem rechten Fuss verwendet wird, und wobei der zweite Ort des zweiten Sondenort-Mittels derart ist, dass beim Betrieb der Sonden-Positioniervorrichtung der zweite Ort neben demselben zweiten Gewebebereich des Fusses ist, und zwar unabhängig davon, ob sie in der ersten Konfiguration mit dem linken Fuss oder in der zweiten Konfiguration mit dem rechten Fuss verwendet wird.

9. System nach Anspruch 7 oder 8, bei welchem die erste und die zweite Arterie aus der folgenden Gruppe ausgewählt werden: dorsalis pedis, posterior tibial, plantar und peroneal desselben Fusses.

10. System nach Anspruch 7, 8 oder 9, bei welchem der erste und der zweite Gewebebereich aus der folgenden Gruppe ausgewählt werden: tibiale Seite vom dorsum des Fusses, die Innenseite des Fusses unterhalb des Knöchels, zwischen dem ersten und dem zweiten Mittelfussknochen, die grosse Zehe und die Ferse des Fusses.

11. System nach einem der vorhergehenden Ansprüche, bei welchem eine zweite Arterie die zweite arterielle Blutzufuhr liefert und die erste und die zweite Arterie Parallelzweig-Arterien von einer dritten Arterie sind, oder bei welchem eine zweite Arterie die zweite arterielle Blutzufuhr liefert und die erste Arterie ein stromabseitiger Zweig der zweiten Arterie ist.

12. System nach Anspruch 8, bei welchem der erste Gewebebereich Teil eines Fusses ist und der zweite Gewebebereich hinter dem Knie desselben Beines ist.

13. Signalverarbeitungseinheit eines Überwachungssystems für post-operative Arterien-Durchgängigkeit zum Überwachen der Durchgängigkeit einer Arterie eines Gliedes, wobei die Einheit aufweist:

eine erste Eingabe zum Empfangen erster Eingabesignale von einer ersten Sonde, welche von einer ersten arteriellen Blutzufuhr zu dem ersten Gewebebereich eines Glieds abhängig sind;
Mittel zum Verarbeiten der ersten Ausgabesignale, um eine erste arterielle Blutzufuhr-Metrik für die erste arterielle Blutzufuhr und eine zweite, unterschiedliche arterielle Blutzufuhr-Metrik für die erste arterielle Blutzufuhr zu erzeugen;
Mittel zum Verwenden einer vorbestimmten Funktion, die als ihre Argumente zumindest die erste und die zweite arterielle Blutzufuhr-Metrik für die erste arterielle Blutzufuhr nimmt,
um eine kombinierte arterielle Durchgängigkeit-Metrik für die erste arterielle Blutzufuhr bereitzustellen;
eine zweite Eingabe zum Empfangen zweiter Eingabesignale von einer zweiten Sonde, welche von einer zweiten arteriellen Blutzufuhr zu einem zweiten Gewebebereich des Glieds abhängig sind;
Mittel zum Verarbeiten der zweiten Ausgabesignale, um eine erste arterielle Blutzufuhr-Metrik für die zweite arterielle Blutzufuhr und eine zweite, unterschiedliche arterielle Blutzufuhr-Metrik für die zweite arterielle Blutzufuhr zu erzeugen;
Mittel zum Verwenden der vorbestimmten Funktion, die als ihre Argumente zumindest die erste und die zweite arterielle Blutzufuhr-Metrik für die zweite arterielle Blutzufuhr nimmt, um eine kombinierte arterielle Durchgängigkeit-Metrik für die zweite arterielle Blutzufuhr bereitzustellen;
Mittel zum Vergleichen der kombinierten arteriellen Durchgängigkeit-Metrik für die erste arterielle Blutzufuhr mit der kombinierten arteriellen Durchgängigkeit-Metrik für die zweite arterielle Blutzufuhr; und
Mittel zum Bestimmen der Durchgängigkeit einer ersten Arterie, welche die erste arterielle Blutzufuhr bereitstellt, unter Verwendung einer Differenz zwischen der kombinierten arteriellen Durchgängigkeit-Metrik für die erste arterielle Blutzufuhr und der kombinierten arteriellen Durchgängigkeit-Metrik für die zweite arterielle Blutzufuhr.

14. Einheit nach Anspruch 13, welche ausserdem eine Signalverarbeitungs-Schaltung zum Verarbeiten der Ausgabe eines Sensors zu einem Hochfrequenz-Signal und einem Niederfrequenz-Signal aufweist.

15. Überwachungsverfahren für post-operative Arterien-Durchgängigkeit zum Überwachen der Durchgängigkeit einer Arterie eines Gliedes, wobei das Verfahren die folgenden Schritte aufweist:

Positionieren einer ersten Sonde neben einem ersten Gewebebereich eines Glieds, das von einer ersten arteriellen Blutzufuhr versorgt wird, so dass sie erste Ausgabesignale erzeugt,
die von der ersten arteriellen Blutzufuhr zu dem ersten Gewebebereich abhängig sind;
Positionieren einer zweiten Sonde neben einem zweiten Gewebebereich des Glieds, das von einer zweiten arteriellen Blutzufuhr versorgt wird, so dass sie zweite Ausgabesignale erzeugt, die von der zweiten arteriellen Blutzufuhr zu dem ersten Gewebebereich abhängig sind;

Verarbeiten der ersten Ausgabesignale, um eine erste arterielle Blutzufuhr-Metrik für die erste arterielle Blutzufuhr und eine zweite, unterschiedliche arterielle Blutzufuhr-Metrik für die erste arterielle Blutzufuhr zu erzeugen;

Kombinieren zumindest der ersten und der zweiten arteriellen Blutzufuhr-Metrik für die erste arterielle Blutzufuhr unter Verwendung einer vorbestimmten Funktion, die als ihre Argumente zumindest die erste und die zweite arterielle Blutzufuhr-Metrik für die erste arterielle Blutzufuhr nimmt, um eine kombinierte arterielle Durchgängigkeit-Metrik für die erste Blutzufuhr bereitzustellen;

Verarbeiten der zweiten Ausgabesignale, um eine erste arterielle Blutzufuhr-Metrik für die zweite arterielle Blutzufuhr und eine zweite, unterschiedliche arterielle Blutzufuhr-Metrik für die zweite arterielle Blutzufuhr zu erzeugen;

Kombinieren zumindest der ersten und der zweiten arteriellen Blutzufuhr-Metrik für die zweite arterielle Blutzufuhr unter Verwendung der vorbestimmten Funktion, die als ihre Argumente zumindest die erste und die zweite arterielle Blutzufuhr-Metrik für die zweite arterielle Blutzufuhr nimmt, um eine kombinierte arterielle Durchgängigkeit-Metrik für die zweite arterielle Blutzufuhr bereitzustellen;

Vergleichen der kombinierten arteriellen Durchgängigkeit-Metrik für die erste arterielle Blutzufuhr mit der kombinierten arteriellen Durchgängigkeit-Metrik für die zweite arterielle Blutzufuhr; und

Bestimmen der Durchgängigkeit einer ersten Arterie, welche die erste arterielle Blutzufuhr bereitstellt, unter Verwendung einer Differenz zwischen der kombinierten arteriellen Durchgängigkeit-Metrik für die erste arterielle Blutzufuhr und der kombinierten arteriellen Durchgängigkeit-Metrik für die zweite arterielle Blutzufuhr.

**Revendications**

1. Système de surveillance de la perméabilité artérielle post-opératoire pour surveiller la perméabilité d'une artère d'un membre, comprenant :

   une première sonde adaptée pour être positionnée à côté d'une première zone de tissu d'un membre pour produire des premiers signaux d'émission dépendant d'une première irrigation sanguine artérielle vers la première zone de tissu ;

   une seconde sonde adaptée pour être positionnée à côté d'une seconde zone de tissu différente du membre pour produire des seconds signaux d'émission différents selon une seconde irrigation sanguine artérielle vers la seconde zone de tissu ; et

   des moyens pour traiter les premiers signaux d'émission pour produire une première mesure de l'irrigation sanguine artérielle pour la première irrigation sanguine artérielle et une seconde mesure différente de l'irrigation sanguine artérielle pour la première irrigation sanguine artérielle ;

   des moyens pour traiter les seconds signaux d'émission pour produire la première mesure de l'irrigation sanguine artérielle pour la seconde irrigation sanguine artérielle et la seconde mesure de l'irrigation sanguine artérielle pour la seconde irrigation sanguine artérielle ;

   des moyens pour utiliser une fonction prédéterminée qui utilise comme arguments au moins la première et la seconde mesure de l'irrigation sanguine artérielle pour la première irrigation sanguine artérielle pour produire une mesure combinée de la perméabilité artérielle pour la première irrigation sanguine artérielle ;

   des moyens pour utiliser la fonction prédéterminée qui utilise comme arguments au moins la première et la seconde mesure de l'irrigation sanguine artérielle pour la seconde irrigation sanguine artérielle pour produire une mesure combinée de la perméabilité artérielle pour la seconde irrigation sanguine artérielle ;

   des moyens pour comparer la mesure combinée de l'irrigation sanguine artérielle pour la première irrigation sanguine artérielle avec la mesure combinée de l'irrigation sanguine artérielle pour la seconde irrigation sanguine artérielle ;

   et

   des moyens pour déterminer la perméabilité d'une première artère qui fournit la première irrigation sanguine artérielle en utilisant une différence entre la mesure combinée de la perméabilité artérielle pour la première irrigation sanguine artérielle et la mesure combinée de la perméabilité artérielle pour la seconde irrigation sanguine artérielle.

2. Système selon la revendication 1, dans lequel la fonction prédéterminée multiplie l'une avec l'autre au moins la première et la seconde mesure de l'irrigation sanguine artérielle pour produire une mesure combinée de la perméabilité artérielle et dans lequel la première mesure de l'irrigation sanguine artérielle et la seconde mesure de l'irrigation sanguine artérielle sont normalisées avant la multiplication.

**3.** Système selon l'une quelconque des revendications précédentes, dans lequel la première mesure de l'irrigation sanguine artérielle et la seconde mesure de l'irrigation sanguine artérielle sont différentes de : la force de signal de l'irrigation sanguine artérielle, le calibre de signal de l'irrigation sanguine artérielle, la fréquence du pouls périphérique, la coloration rouge de la peau et la température de la peau.

**4.** Système selon l'une quelconque des revendications précédentes, dans lequel chaque sonde comprend au moins un premier capteur et un second capteur, le premier capteur fournissant des premiers signaux d'émission utilisés pour produire la première mesure de l'irrigation sanguine artérielle et le second capteur fournissant des premiers signaux d'émission utilisés pour produire la seconde mesure de l'irrigation sanguine artérielle et dans lequel le premier et le second capteur sont différents de : un capteur de pléthysmographe, un capteur de température de la peau et un capteur de coloration de la peau.

**5.** Système selon l'une quelconque des revendications précédentes comprenant en outre un circuit de traitement du signal pour traiter l'émission d'un capteur en un signal de haute fréquence et en un signal de basse fréquence et comprenant en outre des moyens pour former un ratio du signal de haute fréquence sur le signal de basse fréquence.

**6.** Système selon l'une quelconque des revendications précédentes, dans lequel au moins une de la première et de la seconde sonde présente une pluralité de capteurs, le système comprenant en outre des moyens pour traiter les émissions de la pluralité de capteurs pour sélectionner un sous-ensemble de la pluralité de capteurs selon les composants ca des émissions de la pluralité de capteurs et ensuite utilisables pour traiter les émissions uniquement du sous-ensemble sélectionné de capteurs pour évaluer l'irrigation sanguine.

**7.** Système selon l'une quelconque des revendications précédentes, comprenant en outre un appareil de positionnement de sonde comprenant :

un substrat tridimensionnel façonné pour s'adapter à un pied ;
des moyens pour orienter et positionner correctement le substrat à côté d'une partie prédéterminée d'un pied lorsque l'appareil est en cours d'utilisation ;
un premier moyen d'emplacement de sonde en un premier emplacement prédéterminé sur le substrat de sorte que, lorsque l'appareil est en cours d'utilisation, le premier emplacement soit adjacent à la première zone de tissu et à la première artère, et
un second moyen d'emplacement de sonde en un second emplacement prédéterminé sur le substrat de sorte que, lorsque l'appareil est en cours d'utilisation, le second emplacement soit adjacent à la seconde zone de tissu et à une seconde artère.

**8.** Système selon la revendication 7, dans lequel le substrat tridimensionnel peut être inversé de sorte que, dans une première configuration, le substrat soit façonné de manière à s'adapter à un pied gauche et que, dans une seconde configuration, qui est inversée comparativement à la première configuration, le substrat soit façonné de manière à se conformer à un pied droit, le moyen pour orienter et positionner correctement le substrat étant utilisable dans la première configuration et dans la seconde configuration, le premier emplacement du premier moyen d'emplacement de sonde étant tel que, lorsque l'appareil de positionnement de sonde est en cours d'utilisation, le premier emplacement soit adjacent à la même première zone de tissu du pied, qu'il soit utilisé dans la première configuration avec le pied gauche ou dans la seconde configuration avec le pied droit et le second emplacement du second moyen d'emplacement de sonde étant tel que, lorsque l'appareil de positionnement de sonde est en cours d'utilisation, le second emplacement soit adjacent à la même seconde zone de tissu du pied, qu'il soit utilisé dans la première configuration avec le pied gauche ou dans la seconde configuration avec le pied droit.

**9.** Système selon la revendication 7 ou 8, dans lequel la première et la seconde artère sont choisies dans le groupe constitué par : l'artère dorsale du pied, l'artère tibiale postérieure, l'artère plantaire et l'artère péronière du même pied.

**10.** Système selon la revendication 7, 8 ou 9, dans lequel la première et la seconde zone de tissu sont choisies dans le groupe constitué par : le côté tibial du dos du pied, l'intérieur du pied sous la cheville, entre le premier et le second métatarse, le gros orteil et le talon du pied.

**11.** Système selon l'une quelconque des revendications précédentes, dans lequel une seconde artère fournit la seconde irrigation sanguine artérielle et la première et la seconde artère sont des branches artérielles ramifiées parallèles d'une troisième artère ou dans lequel une seconde artère fournit la seconde irrigation sanguine artérielle et la première artère est une ramification en aval de la seconde artère.

**12.** Système selon la revendication 8, dans lequel la première zone de tissu fait partie d'un pied et la seconde zone de tissu se trouve dernière le genou de la même jambe.

**13.** Unité de traitement de signal d'un système de surveillance de la perméabilité artérielle post-opératoire pour surveiller la perméabilité d'une artère d'un membre, l'unité comprenant :

une première entrée pour recevoir les premiers signaux d'entrée issus d'une première sonde qui dépendent d'une première irrigation sanguine artérielle vers une première zone de tissu d'un membre ;

des moyens pour traiter les premiers signaux d'émission pour produire une première mesure de l'irrigation sanguine artérielle et une seconde mesure différente de l'irrigation sanguine artérielle pour la première irrigation sanguine artérielle ;

des moyens pour utiliser une fonction prédéterminée qui utilise comme arguments au moins la première et la seconde mesure de l'irrigation sanguine artérielle pour la première irrigation sanguine artérielle pour produire une mesure combinée de la perméabilité artérielle pour la première irrigation sanguine artérielle ;

une seconde entrée pour recevoir des seconds signaux d'entrée issus d'une seconde sonde qui dépendent d'une seconde irrigation sanguine artérielle vers une seconde zone de tissu du membre ;

des moyens pour traiter les seconds signaux d'émission pour produire une première mesure de l'irrigation sanguine artérielle pour la seconde irrigation sanguine artérielle et une seconde mesure différente de l'irrigation sanguine artérielle pour la seconde irrigation sanguine artérielle ;

des moyens pour utiliser la fonction prédéterminée qui utilise comme arguments au moins la première et la seconde mesure de l'irrigation sanguine artérielle pour la seconde irrigation sanguine artérielle pour produire une mesure combinée de la perméabilité artérielle pour la seconde irrigation sanguine artérielle ;

des moyens pour comparer la mesure combinée de l'irrigation sanguine artérielle pour la première irrigation sanguine artérielle avec la mesure combinée de l'irrigation sanguine artérielle pour la seconde irrigation sanguine artérielle;

et

des moyens pour déterminer la perméabilité d'une première artère qui fournit la première irrigation sanguine artérielle en utilisant une différence entre la mesure combinée de la perméabilité artérielle pour la première irrigation sanguine artérielle et la mesure combinée de la perméabilité artérielle pour la seconde irrigation sanguine artérielle.

**14.** Unité selon la revendication 13 comprenant en outre un circuit de traitement du signal pour traiter l'émission d'un capteur en un signal de haute fréquence et en un signal de basse fréquence.

**15.** Procédé de surveillance de la perméabilité artérielle post-opératoire pour surveiller la perméabilité d'une artère d'un membre comprenant les étapes consistant à :

positionner une première sonde à côté d'une première zone de tissu d'un membre irriguée par une première irrigation sanguine artérielle de sorte qu'elle produise des premiers signaux d'émission qui dépendent de la première irrigation sanguine artérielle vers la première zone de tissu ;

positionner une seconde sonde à côté d'une seconde zone de tissu du membre irriguée par une seconde irrigation sanguine artérielle de sorte qu'elle produise des seconds signaux d'émission qui dépendent de la seconde irrigation sanguine artérielle vers la première zone de tissu ;

traiter les premiers signaux d'émission pour produire une première mesure de l'irrigation sanguine artérielle pour la première irrigation sanguine artérielle et

une seconde mesure différente de l'irrigation sanguine artérielle pour la première irrigation sanguine artérielle ;

combiner au moins la première et la seconde mesure de l'irrigation sanguine artérielle pour la première irrigation sanguine artérielle, en utilisant une fonction prédéterminée qui utilise comme arguments au moins la première et la seconde mesure de l'irrigation sanguine artérielle pour la première irrigation sanguine artérielle, pour produire une mesure combinée de la perméabilité artérielle pour la première irrigation sanguine artérielle ;

traiter les seconds signaux d'émission pour produire une première mesure de l'irrigation sanguine artérielle pour la seconde irrigation sanguine artérielle et

une seconde mesure différente de l'irrigation sanguine artérielle pour la seconde irrigation sanguine artérielle ;

combiner au moins la première et la seconde mesure de l'irrigation sanguine artérielle pour la seconde irrigation sanguine artérielle, en utilisant la fonction prédéterminée qui utilise comme arguments au moins la première et la seconde mesure de l'irrigation sanguine artérielle pour la seconde irrigation sanguine artérielle, pour produire une mesure combinée de la perméabilité artérielle pour la seconde irrigation sanguine artérielle ;

comparer la mesure combinée de la perméabilité artérielle pour la première irrigation sanguine artérielle avec la mesure combinée de la perméabilité artérielle pour la seconde irrigation sanguine artérielle ; et
déterminer la perméabilité d'une première artère qui fournit la première irrigation sanguine artérielle en utilisant une différence entre la mesure combinée de la perméabilité artérielle pour la première irrigation sanguine artérielle et la mesure combinée de la perméabilité artérielle pour la seconde irrigation sanguine artérielle.

Fig. 1

Fig. 2

Fig. 3

Fig. 4

Fig. 5

Fig. 6

Fig. 7

65B

63

65A

72

80

63

62

Fig. 8

2

4  4  4

5  ●  ●  5

4  4  4

5  ●  ●  5

4  4  4

Fig. 9

Fig. 10A

Fig. 10B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **John Allen et al.** Photoplethysmography detection of lower limb peripheral arterial occlusive disease: a comparison of pulse timing, amplitude and shape characteristics. *Physiological Measurement,* October 2005, vol. 26 (5), 811-821